# EUROPÄISCHE PATENTSCHRIFT

(11) **EP 0 280 166 B1**
(45) Veröffentlichungstag und Bekanntmachung des Hinweises auf die Patenterteilung: **14.12.1994**
(21) Anmeldenummer: 88102260.2
(22) Anmeldetag: 17.02.1988
(51) Int. Cl.: C07C 309/03

(54) **Verfahren zur Herstellung von Acyloxialkansulfonaten**
Process for the preparation of acyloxi sulfonates
Procédé de préparation de acyloxi-sulfonates

(30) Priorität: 26.02.1987 DE 3706232
(43) Veröffentlichungstag der Anmeldung: 31.08.1988
(73) Patentinhaber: HOECHST AKTIENGESELLSCHAFT, 65926 Frankfurt am Main (DE)
(72) Erfinder: Reinhardt, Gerd, Dr., D-6233 Kelkheim (Taunus) (DE)

(56) Entgegenhaltungen:
- EP-A- 0 073 626
- DE-A- 1 418 860
- US-A- 3 151 136
- US-A- 3 167 570
- US-A- 4 536 338

## Beschreibung

Acyloxialkansulfonsäuren und deren Salze sind seit langem bekannte Verbindungen mit oberflächenaktiven Eigenschaften, die u.a. als Grundstoff zur Herstellung von Syndet-Seifen Verwendung finden.

Zu ihrer Darstellung sind bereits eine Reihe von Verfahren untersucht worden (J. Am. Oil Chem. Soc. 48, 657 (1971)). In US 1.881.172 und US 2.821.535 ist die Umsetzung von Na-Isethionat mit Fettsäurechloriden beschrieben. Diese Reaktion erfordert den Einsatz von völlig trockenem und feingepulvertem Na-Isethionat; zudem ist die Herstellung der Fettsäurehalogenide aus Fettsäure und Phosphortrichlorid zeit- und kostenintensiv und mit einem Zwangsanfall von phosphoriger Säure verbunden. Bei Verwendung von undestilliertem Säurechlorid ist das Endprodukt meist durch phosphorhaltige Verbindungen verunreinigt, was zu geruchlichen Problemen führen kann. Die Direktkondensation von Na-Isethionat mit Fettsäuren erfordert Reaktionstemperaturen von 220-250°C. Zur Vervollständigung dieser Reaktion muß meist ein Überschuß an Fettsäure eingesetzt werden. Die Reaktion kann ohne Katalysator (DE1.121.045), sowie in Gegenwart von Zinkoxid (US 3,320,292), Zinksalzen organischer Säuren (US 4,405,526), Borsäure (US 2,923,724) oder phosphorhaltiger Säuren (US 3,004,049) durchgeführt werden. Bei Verwendung von gasförmigem Chlorwasserstoff als Katalysator ist eine Direktveresterung bereits bei 180°C möglich (US 3,167,570). Nach US 3,151,136 gelingt die Veresterung von freier Isethionsäure mit Fettsäure bereits bei 130°C, jedoch bereiten die technische Herstellung von freier Isethionsäure aus deren leicht zugänglichen Salzen sowie die Neutralisation des Kondensationsproduktes Schwierigkeiten.

Aufgabe der vorliegenden Erfindung ist es, ein auch im großtechnischen Maßstab leicht durchführbares Verfahren zur Herstellung von Salzen von Acyloxialkansulfonsäuren aufzuzeigen, bei dem ausgehend Von Salzen von Hydroxialkansulfonsäuren und Fettsäuren eine Direktkondensation schon unter milden Reaktionsbedingungen durchgeführt werden kann.

Überraschenderweise wurde nun gefunden, daß eine Direktkondensation von Salzen der Hydroxialkansulfonsäuren mit Fettsäuren bereits bei Temperaturen unterhalb von 150°C in kurzen Reaktionszeiten durchgeführt werden kann, wenn dabei wäßrige Salzsäure als Katalysator eingesetzt wird. Dies konnte nicht erwartet werden, da bekannt ist, daß Acyloxialkansulfonate in Gegenwart wäßriger Säuren extrem leicht verseifen.

Gegenstand der Erfindung ist daher ein Verfahren zur Herstellung von Salzen von Acyloxialkansulfonsäuren der Formel 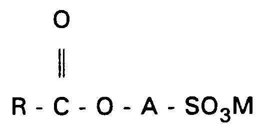
wobei R C₇-C₂₁-Alkyl oder C₇-C₂₁-Alkenyl, A C₂-C₄-Alkylen und M ein Alkali- oder Erdalkalimetall-Atom bedeuten, durch Veresterung von Carbonsäuren der allgemeinen Formel R-CO₂H, wobei R die zuvor angegebene Bedeutung hat, mit Salzen von Hydroxialkansulfonsäuren der Formel HO-A-SO₃M, wobei A und M die zuvor angegebene Bedeutung haben, das dadurch gekennzeichnet ist, daß eine Mischung aus der Säure, einem Salz der Hydroxialkansulfonsäure und wäßriger Salzsäure auf 70 - 145°C erhitzt wird, und das Reaktionswasser sowie die wäßrige Salzsäure während der Reaktion abdestilliert wird.
Ebenfalls Gegenstand der Erfindung ist ein Verfahren, das dadurch gekennzeichnet ist, daß eine Mischung aus der Säure, einem Salz der Hydroxialkansulfonsäure und Wasser auf 70 - 145°C erhitzt wird, gasförmige Salzsäure in die Mischung eingeleitet wird, und das Reaktionswasser sowie die wäßrige Salzsäure während der Reaktion abdestilliert wird. Wahrscheinlich spaltet dabei die Salzsäure einen Teil des Hydroxialkansulfonats in die freie Hydroxialkansulfonsäure und das Metallchlorid. Nach der Veresterung wird dann die gebildete Acyloxialkansulfonsäure durch das Metallchlorid unter Freisetzung von Chlorwasserstoff neutralisiert. Das im Reaktionsmedium vorhandene Wasser fördert dabei die bessere Durchmischung der Reaktionskomponenten und ermöglicht dadurch eine niedrige Reaktionstemperatur sowie kurze Reaktionszeiten.

Nach diesem Verfahren wird eine Mischung aus festem Metall-Hydroxialkansulfonat, Fettsäure und wäßriger Salzsäure bzw. eine Mischung, die aus Hydroxialkansulfonat-Lösung und Fettsäure durch Einleiten von gasförmigem Chlorwasserstoff erhalten wird, zur Initierung der Reaktion auf 70-120°C, vorzugsweise 90-110°C, erwärmt und zur Vervollständigung der Reaktion unter Temperatursteigerung bis auf 110-145°C, vorzugsweise 120-135°C, unter intensivem Rühren erhitzt. Dabei werden - zweckmäßig unter reduziertem Druck - die wäßrige Salzsäure und das Reaktionswasser aus dem Reaktionsgemisch entfernt. Im Verlaufe der Umsetzung verfestigt sich das anfangs dünnflüssige Reaktionsgemisch zu einer Paste, aus der schließlich eine weiße feste Masse entsteht, aus der das Produkt durch Zerkleinern in Pulverform erhalten werden kann. Es ist auch möglich, das Reaktionsprodukt in Wasser zu lösen und anschließend durch Sprühtrocknung zu isolieren. Dabei wird im Produkt verbliebener Chlorwasserstoff vollständig entfernt. Das Endprodukt ist pH-neutral oder schwach sauer und kann, falls erforderlich, durch Zugabe von Alkali- oder Erdalkalimetallcarbonaten oder -hydrogencarbonaten im pH-Wert korrigiert werden. Der Natriumchloridgehalt des Endproduktes ist im allgemeinen kleiner als 0,5 %.

In einer besonderen Ausführungsform dieses Verfahrens kann dem Reaktionsgemisch vor oder während der Kondensation freie Hydroxialkansulfonsäure in einer Konzentration bis zu 5 Mol % zugesetzt werden. Desweiteren können bleichende Zusätze zum Reaktionsgemisch zugegeben werden.

Typische Hydroxialkansulfonsäuren, die verestert werden können, sind Monohydroxialkansulfonsäuren mit 2 bis 4 Kohlenstoffatomen, insbesondere gradkettige, aber auch verzweigtkettige 2-Hydroxialkansulfonsäuren, z.B. 2-Hydroxiethansulfonsäure (Isethionsäure), 2-Hydroxipropansulfonsäure und Hydroxibutansulfonsäure. Die Hydroxialkansulfonsäure wird in Form ihrer Alkali- oder Erdalkalisalze, vorzugsweise als Natriumsalz eingesetzt. Dabei kann das Salz sowohl in festem Zustand als auch als wäßrige Lösung eingesetzt werden. Bei Verwendung von trocknem Hydroxialkansulfonat wird wäßrige Salzsäure zum Reaktionsgemisch zugegeben, beim Einsatz einer Hydroxialkansulfonat-Lösung wird die wäßrige Salzsäure zweckmäßigerweise durch Einleiten von gasförmigem Chlorwasserstoff in das Reaktionsgemisch bei 10-145°C, vorzugsweise 20-110°C hergestellt. Der Gehalt an Chlorwasserstoff im Reaktionsgemisch zu Beginn der Kondensation sollte 0,3 bis 2 Mol, vorzugsweise 0,6 bis 1,3 Mol, bezogen auf eingesetztes Hydroxialkansulfonat betragen.

Typische Carbonsäuren, die eingesetzt werden können, sind alle geradkettigen oder verzweigten Carbonsäuren, insbesondere höhere Fettsäuren, gesättigt oder ungesättigt, mit 8 bis 22 Kohlenstoffatomen wie z.B. Octansäure, Laurinsäure, Palmitinsäure, Stearinsäure oder Mischungen davon wie z.B. Tallölfettsäure, Talgfettsäure oder Kokosfettsäure. Es werden 0,5 bis 2 Mol, vorzugsweise 0,8 bis 1,2 Mol Fettsäure pro Mol Hydroxialkansulfonat eingesetzt.

Die Reaktionszeit beträgt 30 min bis 10 h, vorzugsweise jedoch 1 bis 4 h.

Die Vorteile der hier beschriebenen Direktkondensation sind neben hohen Umsetzungsgraden niedrige Reaktionstemperaturen und kurze Reaktionszeiten. Zudem ist das Produkt frei von zink-, bor- oder phosphorhaltigen Fremdstoffen, die normalerweise bei Direktveresterungen als Katalysatoren eingesetzt werden.

In den folgenden Beispielen verhalten sich Gew.-Teile zu Vol.-Teilen wie kg zu dcm³, % Angaben beziehen sich - wenn nichts anderes angegeben - immer auf das Gewicht. Unter dem Begriff WS-Gehalt ist in den nachfolgenden Beispielen der Anteil an waschaktiver Substanz im Produkt zu verstehen, der durch 2-Phasen-Titration nach EPTON (Nature 160, 759 (1947)) bestimmt wird.

### Beispiel 1

148 Teile Na-Isethionat, 222 Teile gehärteter Kokosfettsäure und 100 Teile konzentrierte wäßrige Salzsäure werden gemischt und 30 min auf 100-110°C erhitzt. Anschließend wird die Temperatur innerhalb 150 min auf 130°C erhöht. Während dieser Zeit wird wäßrige Salzsäure durch Abdestillation unter vermindertem Druck (40 mm Hg) aus dem Reaktionsgemisch entfernt. Nach dem Abkühlen und Zerkleinern erhält man in quantitativer Ausbeute ein weißes Pulver mit einem WS-Gehalt von 88,6 %.

### Beispiel 2

148 Teile Na-Isethionat, 222 Teile gehärteter Kokosfettsäure, 5 Teile Isethionsäure und 100 Teile konzentrierte wäßrige Salzsäure werden wie in Beispiel 1 zur Reaktion gebracht. Nach beendeter Reaktion wird das weiße Pulver durch Verkneten mit Natriumhydrogencarbonat auf pH 5,5 eingestellt. Der WS-Gehalt beträgt 89,4 %.

### Beispiel 3

Es wird nach Beispiel 1 verfahren, es werden jedoch 85 Teile konz. wäßrige Salzsäure eingesetzt.
Man erhält ein weißes Pulver mit einem WS-Gehalt von 86,2 %.

### Beispiel 4 148 Teile Na-Isethionat, 200 Teile Laurinsäure und 110 Teile konzentrierte wäßrige Salzsäure werden analog Beispiel 1 zur Reaktion gebracht. Man erhält ein weißes Pulver mit einem WS-Gehalt von 90,8 %.

### Beispiel 5

296 Teile einer technischen 50%igen Na-Isethionat-Lösung und 222 Teile gehärteter Kokosfettsäure werden auf 90-110°C erhitzt. 30 min lang wird HCL-Gas so in die Lösung eingeleitet, daß der HCl-Gehalt ca. 1 Mol beträgt. Anschließend wird das Gemisch unter reduziertem Druck (40 mm) 2 h auf 120°C, dann zur Vervollständigung der Reaktion 1 h auf 130°C erhitzt. Man erhält ein weißes Pulver mit einem WS-Gehalt von 87,7 %.

## Patentansprüche

1. Verfahren zur Herstellung von Acyloxisulfonaten der Formel
R - C(O) - O - A - SO₃M
wobei R C₇-C₂₁-Alkyl oder C₇-C₂₁-Alkenyl, A C₂-C₄-Alkylen und M ein Alkali- oder Erdalkalimetall-Atom bedeuten, durch Veresterung von Carbonsäuren der allgemeinen Formel R - CO₂H, wobei R die zuvor angegebene Bedeutung hat, mit Salzen von Hydroxialkansulfonsäuren der Formel HO - A - SO₃M, wobei A und M die zuvor angegebene Bedeutung haben, dadurch gekennzeichnet, daß eine Mischung aus der Säure, einem Salz der Hydroxialkansulfonsäure und wäßriger Salzsäure auf 70 - 145°C erhitzt wird, und das Reaktionswasser sowie die wäßrige Salzsäure während der Reaktion abdestilliert wird.

2. Verfahren zur Herstellung von Acyloxisulfonaten der Formel
R - C(O) - O - A - SO₃M
wobei R C₇-C₂₁-Alkyl oder C₇-C₂₁-Alkenyl, A C₂-C₄-Alkylen und M ein Alkali- oder Erdalkalimetall-Atom bedeuten, durch Veresterung von Carbonsäuren der allgemeinen Formel R - CO₂H, wobei R die zuvor angegebene Bedeutung hat, mit Salzen von Hydroxialkansulfonsäuren der Formel HO - A - SO₃M, wobei A und M die zuvor angegebene Bedeutung haben, dadurch gekennzeichnet, daß eine Mischung aus der Säure, einem Salz der Hydroxialkansulfonsäure und Wasser auf 70 - 145°C erhitzt wird, gasförmige Salzsäure in die Mischung eingeleitet wird, und das Reaktionswasser sowie die wäßrige Salzsäure während der Reaktion abdestilliert wird.

3. Verfahren nach Anspruch 1 oder 2, dadurch gekennzeichnet, daß die Mischung zunächst auf 70 - 120°C, vorzugsweise auf 90 - 110°C erhitzt wird und anschließend auf 110 - 145°C, vorzugsweise 120 - 135°C erhitzt wird.

4. Verfahren nach einem der Ansprüche 1 bis 3, dadurch gekennzeichnet, daß die Reaktion unter vermindertem Druck durchgeführt wird.

5. Verfahren nach einem der Ansprüche 1 bis 4, dadurch gekennzeichnet, daß dem Reaktionsgemisch vor oder während der Reaktion 1 - 5 Mol.-% Isethionsäure zugesetzt werden.

## Claims

1. A process for the preparation of an acyloxysulfonate of the formula
R - C(O) - O - A - SO₃M
in which R is C₇-C₂₁-alkyl or C₇-C₂₁-alkenyl, A is C₂-C₄-alkylene and M is an alkali metal or alkaline earth metal atom, by esterification of a carboxylic acid of the formula R - CO₂H, in which R is as defined above, with a salt of a hydroxyalkanesulfonic acid of the formula HO - A - SO₃M, in which A and M are as defined above, which comprises heating a mixture of the acid, a salt of the hydroxyalkanesulfonic acid and aqueous hydrochloric acid at 70 - 145°C and distilling off the water of reaction and the aqueous hydrochloric acid during the reaction.

2. A process for the preparation of an acyloxysulfonate of the formula
R - C(O) - O - A - SO₃M
in which R is C₇-C₂₁-alkyl or C₇-C₂₁-alkenyl, A is C₂-C₄-alkylene and M is an alkali metal or alkaline earth metal atom by esterification of a carboxylic acid of the formula R - CO₂H, in which R is as defined above, with a salt of a hydroxyalkanesulfonic acid of the formula HO - A - SO₃M, in which A and M are as defined above, which comprises heating a mixture of the acid, a salt of the hydroxyalkanesulfonic acid and water at 70 - 145°C, passing gaseous hydrochloric acid into the mixture and distilling off the water of reaction and the aqueous hydrochloric acid during the reaction.

3. A process as claimed in claim 1 or 2, wherein the mixture is heated first at 70 - 120°C, preferably at 90 - 110°C, and then at 110 - 145°C, preferably 120 - 135°C.

4. A process as claimed in one of claims 1 to 3, wherein the reaction is carried out under reduced pressure.

5. A process as claimed in one of claims 1 to 4, wherein 1 - 5 mol% of isethionic acid is added to the reaction mixture before or during the reaction.

## Revendications

1. Procédé de production d'acyloxy sulfonates de formule :
R - C (O) - O - A - SO₃M
(dans laquelle R représente un groupe alkyle en C₇ à C₂₁ ou un groupe alcényle en C₇ à C₂₁, A représente un groupe alkylène en C₂-C₄ et M représente un atome de métal alcalin ou alcalino terreux) par estérification d'acides carboxyliques de formule générale R-CO₂H (dans laquelle R a le sens précité) avec des sels d'acides hydroxy alcane sulfoniques de formule HO-A-SO₃M (dans laquelle A et M ont le sens précité), procédé caractérisé en ce qu'on soumet un mélange de l'acide, d'un sel d'acide hydroxy alcane sulfonique et de l'acide chlorhydrique aqueux à du chauffage jusqu'à 70-145°C et l'on chasse par distillation, pendant la réaction, l'eau résultant de la réaction ainsi que l'acide chlorhydrique aqueux.

2. Procédé pour produire des acyloxy sulfonates de formule :
R - C (O) - O - A - SO₃M
(dans laquelle R représente un groupe alkyle en C₇ à C₂₁ ou alcényle en C₇ à C₂₁. A représente un groupe alkylène en C₂ à C₄ et M représente un atome de métal alcalin ou alcalino terreux) par estérification d'acides carboxyliques de formule R-CO₂H (dans laquelle R a le sens précité) avec des sels d'acides hydroxy alcane sulfoniques de formule HO-A-SO₃M (dans laquelle A et M ont le sens précité), procédé caractérisé en ce qu'on chauffe un mélange de l'acide, d'un sel de l'acide hydroxy alcane sulfonique et de l'eau à 70-145°C, on fait passer du gaz chlorhydrique dans le mélange et l'on chasse par distillation, pendant la réaction, l'eau résultant de la réaction ainsi que l'acide chlorhydrique aqueux.

3. Procédé selon la revendication 1 ou 2, caractérisé en ce qu'on chauffe le mélange tout d'abord à 70-120°C, avantageusement à 90-110°C puis à 110-145°C, avantageusement à 120°C-135°C.

4. Procédé selon l'une des revendications 1 à 3, caractérisé en ce qu'on effectue la réaction sous pression réduite.

5. Procédé selon l'une quelconque des revendications 1 à 4, caractérisé en ce qu'on ajoute au mélange réactionnel, avant ou pendant la réaction, 1 à 5 moles % d'acide iséthionique.
